# EUROPEAN PATENT SPECIFICATION

(11) **EP 2 666 368 B1**
(45) Date of publication and mention of the grant of the patent: **01.02.2017**
(21) Application number: 13180900.6
(22) Date of filing: 27.07.2009
(51) Int. Cl.: A23L 29/20, A23P 20/10, A23G 4/06, A23G 3/34, A23G 3/42

(54) **Use of gum arabic replacement in panning, confections and adhesion applications**
Verwendung von Gummiarabikumersatz bei Schwenk-, Süßwaren- und Haftanwendungen
Utilisation de succédané de gomme arabique pour cuisson, confiseries et applications d'adhésion

(30) Priority: 25.07.2008 US 83808 P
(43) Date of publication of application: 27.11.2013
(62) Divisional of application: 09790853.7
(73) Proprietor: Tic Gums, Inc., Belcamp, MD 21017 (US)
(72) Inventor: Nieto, Marceliano B., Abingdon, MD Maryland 21009 (US); Andon, Greg, Belcamp, MD Maryland 21017 (US)
(74) Representative: Müller Schupfner & Partner Patent- und Rechtsanwaltspartnerschaft mbB

(56) References cited:
- EP-A1- 0 797 925
- WO-A1-02/19987
- WO-A1-2008/016940
- WO-A2-2007/112077
- GB-A- 2 115 672
- US-A- 3 870 527
- US-A- 5 545 417
- US-A1- 2002 113 632
- US-A1- 2004 037 922
- US-A1- 2004 180 110

## Description

### BACKGROUND OF THE INVENTION

### Field of the Invention

This disclosure relates generally to gum arabic replacements targeted but not limited to their use in the application of panning, confections, and food adhesion to achieve various functionalities derived from gum Arabic, such as: sugar binding and strengthening of the shell of chewing gum and panned peanuts, chocolate or gum balls; as sealant and oxygen barrier for oil-containing centers such as various nuts, chocolate and malt balls; as texture modifier hardening agent for pastilles and hard candies; as polish to provide a shiny coat for panned products, cereals, tablets and other products and as an adhesive for granola bars, cereal clusters and seeds.

### Discussion of the Related Art

Gum arabic is the dried exudate obtained from various species of trees of the genus Acacia of the Leguminosae family in the tropical and semi-tropical areas of the world. Gum arabic has multiple uses in food, pharmaceutical and industrial applications. Currently there are two species of Acacia that are commercially used: Acacia Senegal and Acacia Seyal for food and pharmaceutical applications. However, for industrial applications, specifically in lithography, another tree exudate called Combretum is becoming the standard for replacement. The major producing countries of these species are in the Sahelian region of Africa including the Republic of the Sudan, Chad, Eritrea, Nigeria and neighboring countries. Shortage of gum arabic is not uncommon due to political turmoil in this region of Africa.

The need for gum arabic for food and pharmaceutical applications is very significant. For panning alone, an estimate of worldwide demand for gum arabic is about 20 million pounds per year. In recent years, the supply of gum arabic has been very volatile and unpredictable due to the political instability in the region of Africa where it is sourced. Accordingly, the risk of not having a sufficient supply of gum arabic has become a growing concern.

Gum arabic is a high MW polysaccharide with an unusually low viscosity, behaving as Newtonian liquid up to 35% concentration; it dissolves at concentrations of 55%-60% to make a thick syrup. This low viscosity is attributed to its structural branching, which makes it a globular molecule. This branching prevents micelle formation, unlike linear gums, minimizing intermolecular hydrogen bonding when it is hydrated in water. As a result, it forms a weak film. Its solution gets tacky at high concentrations but gives brittle texture when dried. It forms a shiny film when cast on a surface that cracks in a unique pattern. It has an emulsifying property attributed to the presence of protein that is covalently linked to some portion of the polysaccharide fraction forming a very high MW glycoprotein complex of greater than 2 million Daltons.

Gum arabic is also used in hard panning of chewing gums and chocolate panned confections to bind and strengthen the shell coat that is made up of sugar or sugar alcohols. It is added to the syrup recipe for spraying or ladling onto the centers. Gum arabic is either used only during the initial stage of the panning process for cost reasons, or in all the charges of syrup, especially in sugar-free formulations to help build a strong layer of shell around the chewing gum centers. The need for a good binder in coating and engrossing syrups is a direct result of scale-up or the desire to use larger coating pans to increase capacity, and this results in more breakage of the shell coat as the centers tumble in the pan. In many other instances, cracking happens during packaging, transport of the finished product and when consumers shake the packaging container. Even in regular sugar panning, the need to strengthen the shell during the coating process becomes critical because of the use of large pan coaters. Maltodextrin, a cheaper substitute for binding the sugar in the regular syrup coating, may work satisfactorily when coating pans and batch sizes are small; however, it fails as a gum arabic replacement when using larger coating pans and batch sizes due to weaker and crumblier crystals.

Acacia Senegal is a premium grade of gum arabic that is a natural emulsifier mainly used in beverage emulsions, savory flavor emulsions and spray dried flavors; Acacia Seyal is a grade of gum arabic that has limited emulsifying properties but has lower cost structure. Acacia Seyal has replaced Senegal in many applications where emulsification is secondary or not critical. Replacements for Acacia Senegal in beverage emulsions have been developed and commercialized, such as various emulsifying starches and the OSA-modified gum Acacia made from Acacia Seyal as disclosed in U.S. Patent No. 6,455,512. In flavor encapsulation/spray dried flavors, straight Acacia Seyal, blends containing Acacia Seyal and modified gum arabic and blends containing emulsifying starches with maltodextrin have partly replaced Acacia Senegal. Embodiments of the invention differ from these current replacements in composition of ingredients that were evaluated and chosen based on their contribution to matching key properties of gum arabic in various applications, or in some cases, improving the functionality of the replacements over that of gum arabic while reducing or matching cost.

In other applications such as panning and confections, the main replacement for gum arabic is the use of Acacia Seyal in place of Acacia Senegal, a practice that will not solve the impending shortage problem with gum arabic. Hard panning or coating of comestible or pharmaceutical compositions that use sugars, such as, sucrose, dextrose, fructose or glucose syrups, or use sugar-free compositions containing maltitol, erythritol, sorbitol, xylitol, mannitol or hydrogenated starch hydrolysates almost always requires the use of gum arabic for binding, strengthening of the shell or sealing. The process for applying the coatings to the cores generally consists of tumbling the cores in a rotating pan at a desired speed and temperature, applying multiple charges of the coating in a liquid form and drying the coating in between coat application. The coating process is repeated until the shell coat has the desired thickness, about 30-33% of the product weight. Variations of this process have been developed.

EP 0 797 925 relates to a process for encapsulating solid particles with a
protein. This document discloses that the concentration of the polysaccharide in the aqueous medium may be from 0.1 to 10% by weight. The document also teaches that the polysaccharide may be pectin such as high methoxy pectin, an alginate, arabic gum, locust bean gum or kappa-, lambda-, or iota-carrageenan. US 2004/0037922 relates to a composition for coating foodstuff which comprises a first polysaccharide chosen from the group consisting of alginate, pectin, carrageenan and a second polysaccharide which can be cellulose, methylcellulose, hydroxypropylcellulose, methylcellulose and galactomannans (such as e.g. guar gum, tara gum and carob meal). WO 2007/112077 relates to methods and products for forming an anti-scuff coating on solid chocolate, wherein the coating composition comprises film forming agents such as xanthan gum, gum arabic, guar gum, locust bean gum, agar, alginates, gum ghatti, gum karaya, gum tragacanth, chitosan, carrageenans, methylcellulose, carboxymethylcellulose, hydroxypropyl methylcellulose, ethylcellulose, hydroxypropyl cellulose, pectin, gelatin, modified starch, dextrin, zein and whey protein. Further, this document teaches that the composition comprises 0.01 to 40% by weight of the first film forming agent. GB 2 115 672 relates to a method for applying sugarless coating to chewing gum and confections and discloses adhesion binders including gum arabic, xanthan gum, gum tragacanth, tapioca dextrin, or modified food starch, with gum arabic being preferred and that the binder may be present in a range from 5 to 30%. US 3,870,527 relates to starch-based gum replacements for gum Arabic and discloses starch-based gums, which are particularly useful in lithography. US 2004/0180110 relates to an edible composition and discloses a film forming agent selected from pullulan, hydroxymethylmethyl cellulose, hydroxyethyl cellulose, hydroxypropyl cellulose, polyvinyl pyrrolidone, carboxymethyl cellulose, polyvinyl alcohol, sodium alginate, polyethylene glycol, xanthan gum, tragacanth gum, guar gum, acacia gum, arabic gum, polyacrylic acid, methylmethacrylate colpolymer etc.. US 2002/0113632 relates to chewing gum products that include an outer shell or coating and discloses panning modifiers including gum arabic, maltodextrins, corn syrup, gelatin, cellulose type materials, starch and modified starches, vegetable gums like alginates, locust bean gum, guar gum and gum tragacanth, insoluble carbonates and talc. US 5,545,417 relates to chewing gum products and teaches panning modifiers including gum arabic, maltodextrins, corn syrup, gelatin, cellulose type materials, starch and modified starches, vegetable gums like alginates, locust bean gum, guar gum and gum tragacanth, insoluble carbonates and talc. WO 02/19987 relates to an edible dry-powder film coating composition and discloses gum acacia, hydroxypropyl methylcellulose, methyl cellulose, polyethylene glycol and starch. WO2008/016940 relates to chewing gum products and teaches panning modifiers including gum arabic, maltodextrins, corn syrup, gelatin, cellulose type materials, starch and modified starches, vegetable gums like alginates, locust bean gum, guar gum and gum tragacanth, insoluble carbonates and talc. In edible film and glitter applications where viscosity, clarity and specific film cracking pattern and adhesion/release from the casting belt are critical, gum arabic is still the sole gum material currently used for this purpose.

Due to the uniqueness of gum arabic, related arts to replace it in various uses such as food, pharmaceutical and industrial applications are scarce. Therefore, there is a need for a replacement for gum arabic.

### SUMMARY OF THE INVENTION

Accordingly, the invention is directed towards gum arabic replacement, partial replacement or extending replacements and use of the same in various applications, e.g., food industry, pharmaceutical industry, and others, that substantially obviates one or more of the problems due to limitations and disadvantages of the related art.

An advantage of the invention is to provide gum arabic replacement using locally available materials that will reduce reliance on imported gum arabic.

Another advantage of the invention is to provide gum arabic replacement with competitive or lower cost structure than gum arabic.

Other advantages of the present invention are the added improvement in functionality in certain applications such as the whiter color, faster drying when used in panning and better film barrier and protection against oxidation.

Additional features and advantages of the invention will be set forth in the description which follows, and in part will be apparent from the description, or may be learned by practice of the invention. These features and other advantages of the invention will be realized and attained by the structure particularly pointed out in the written description and claims hereof as well as the appended drawings.

The invention is directed towards a method of preparing a gum arabic replacement, partial replacement or extending composition, for use in panning, candy, coating and food adhesion applications. The method includes blending a strong (key) film former selected from the group consisting of carrageenan, pectin, alginate, gellan, agar, konjac, xanthan, tara, fenugreek, cellulose gum, methyl cellulose, hydroxypropyl methylcellulose (HPMC), propylene glycol alginate, or a combination thereof between approximately 0.1% and approximately 10% of a total composition with; a low viscosity cracking agent selected from the group consisting of maltodextrin, monosaccharides, disaccharides, oligosaccharides, larch gum, polydextrose, or a combination thereof; a tackiness modifier selected from the group consisting of starch, guar gum, locust bean gum, or a combination thereof; a fast-crystallizing agent selected from the group consisting of inulin, erythritol, low DE maltodextrin, lactose, or a combination thereof, .
wherein the composition has a viscosity at concentrations of about 35-40°Brix of being about 10 cP to about 2000 cP at 25 °C and a film cast at 5 mils to 10 mils cracks into flakes on drying that lies between that of materials that can generate continuous films and materials that crystallize and do not form films at all.

It is to be understood that both the foregoing general description and the following detailed description are exemplary and explanatory and are intended to provide further explanation of the invention as claimed.

### BRIEF DESCRIPTION OF THE DRAWINGS

The accompanying drawings, which are included to provide a further understanding of the invention and are incorporated in and constitute a part of this specification, illustrate embodiments of the invention and together with the description serve to explain the principles of the invention.

In the drawings:
FIG. 1A illustrates a film cracking pattern of Acacia Seyal gum arabic according to a reference example of the invention;
FIG. 1B illustrates a film cracking pattern of Acacia Senegal gum arabic according to a reference example of the invention;
FIG. 2 illustrates a viscosity profile of gum arabic compositions in FIGS. 1A and 1B;
FIG. 3A illustrates a CMC or cellulose gum film according to another example of the invention;
FIG. 3B illustrates a methylcellulose gum film according to another example of the invention;
FIG. 3C illustrates a pectin film according to another example of the invention;
FIG. 3D illustrates an agar film according to another example of the invention;
FIG. 3E illustrates a sodium alginate film according to a reference example of the invention;
FIG. 3F illustrates a locust bean gum film according to another example of the invention;
FIG. 3G illustrates a xanthan gum film according to another example of the invention;
FIG. 3H illustrates a carrageenan film according to another example of the invention;
FIG. 4A illustrates an inulin film according to another example of the invention;
FIG. 4B illustrates a larch gum film according to another example of the invention;
FIG. 4C illustrates a low viscosity starch film according to another example of the invention;
FIG. 4D illustrates a maltodextrin film according to another example of the invention;

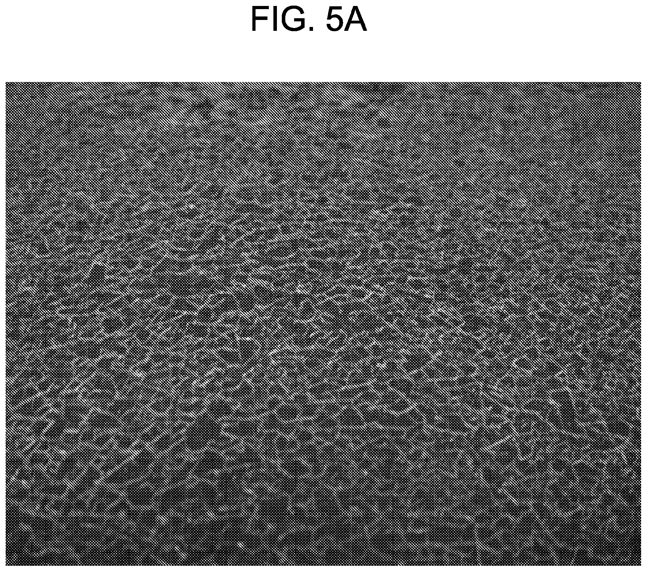

FIG. 5A illustrates a film cracking pattern of Replacement 1 Std. according to another example of the invention;

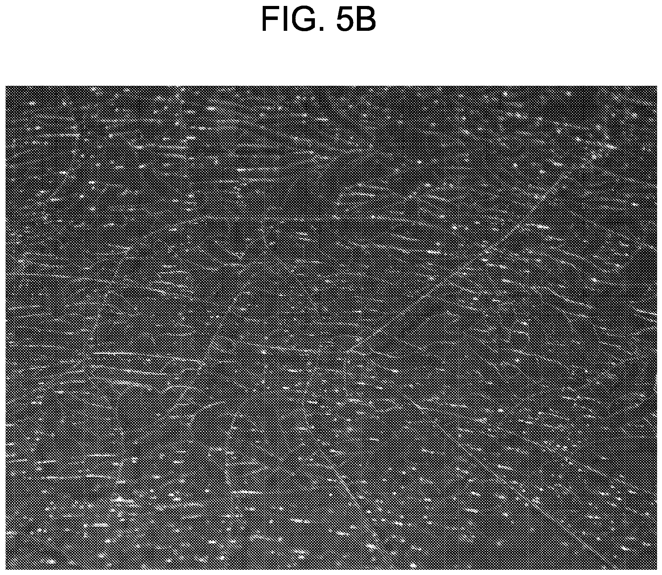

FIG. 5B illustrates a film cracking pattern of Replacement 1 All Natural according to another example of the invention;

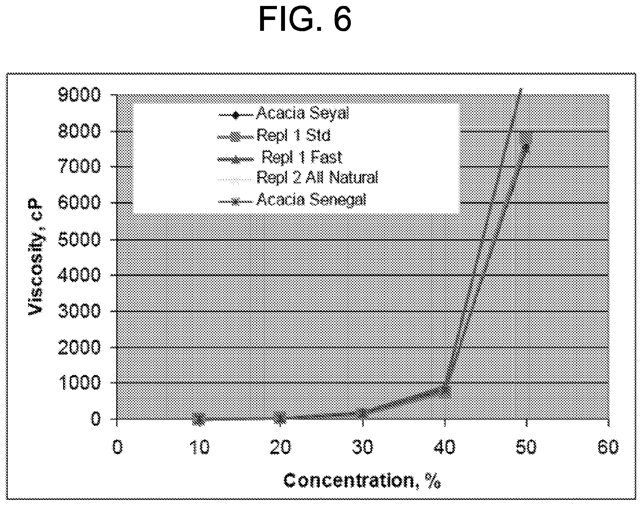

FIG. 6 illustrates viscosity profiles according to another example of the invention;

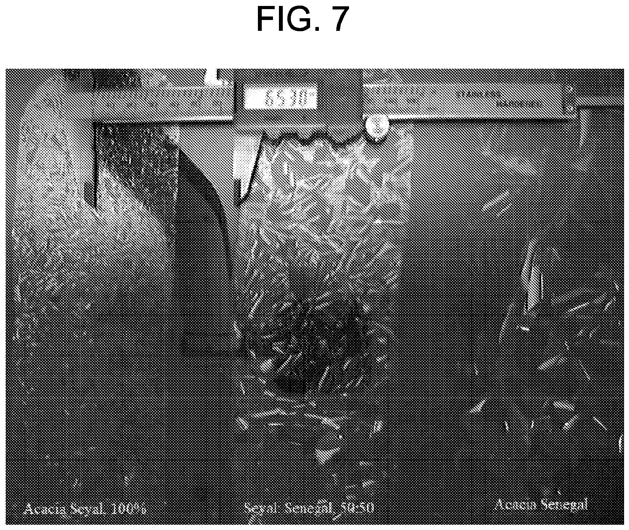

FIG. 7 illustrates three different films having different flake patterns according to another example of the invention;

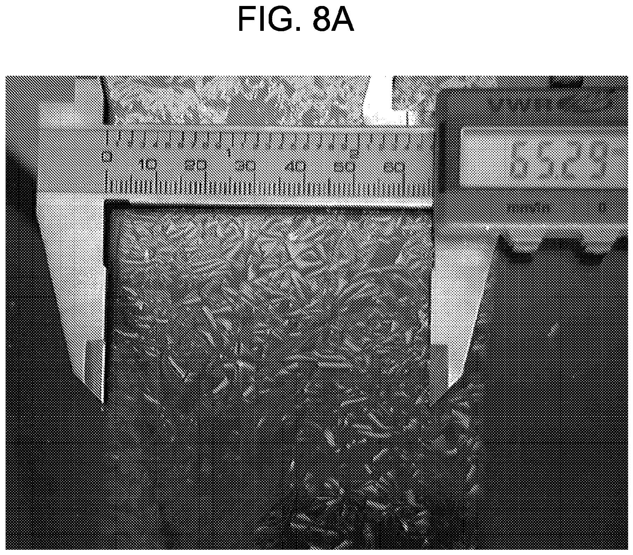

FIG. 8A illustrates an Acacia Seyal gum arabic film having a flake pattern according to another example of the invention;

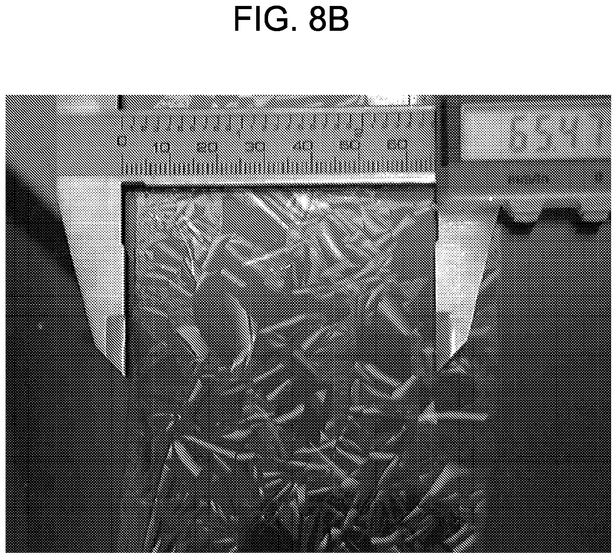

FIG. 8B illustrates a 50:50 blend of Acacia Seyal:Acacia Senegal gum arabic film having a flake pattern according to a reference example of the invention;

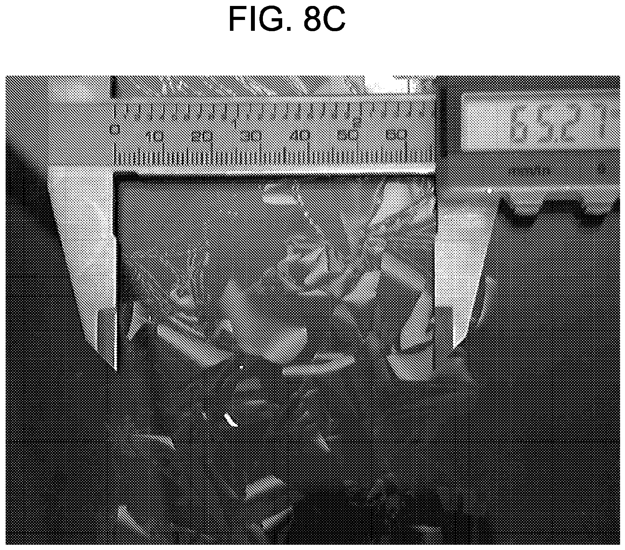

FIG. 8C illustrates an Acacia Senegal film having a flake pattern according to a reference example of the invention;

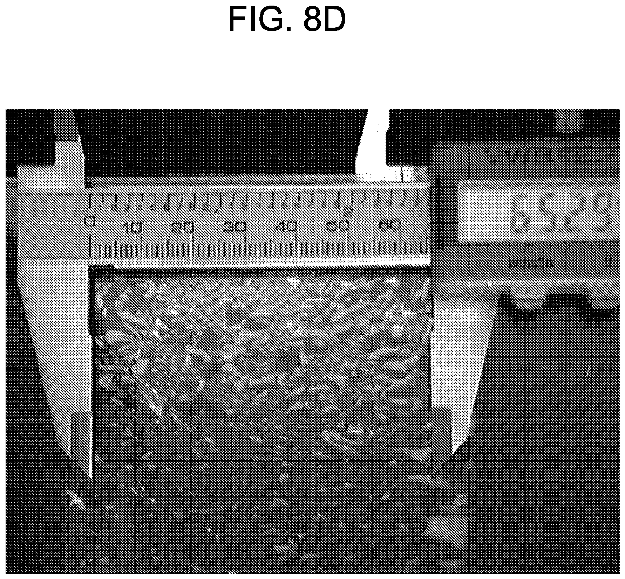

FIG. 8D illustrates an Acacia Seyal Replacement 1 Std. film having a flake pattern according to a reference example of the invention;

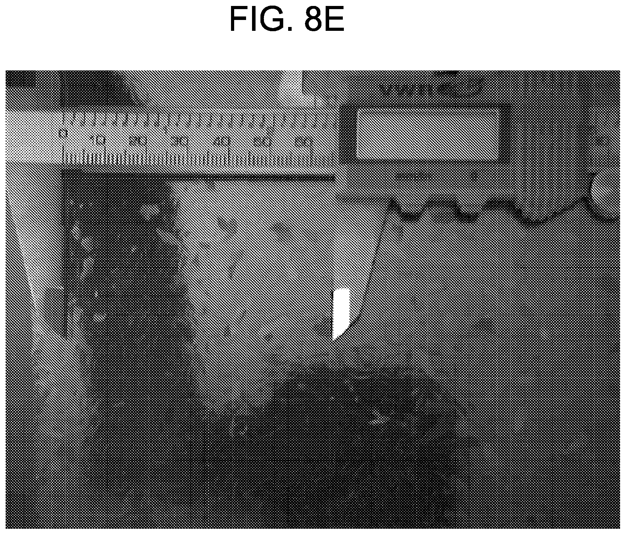

FIG. 8E illustrates an Acacia Seyal film having a flake pattern according to a reference example of the invention;

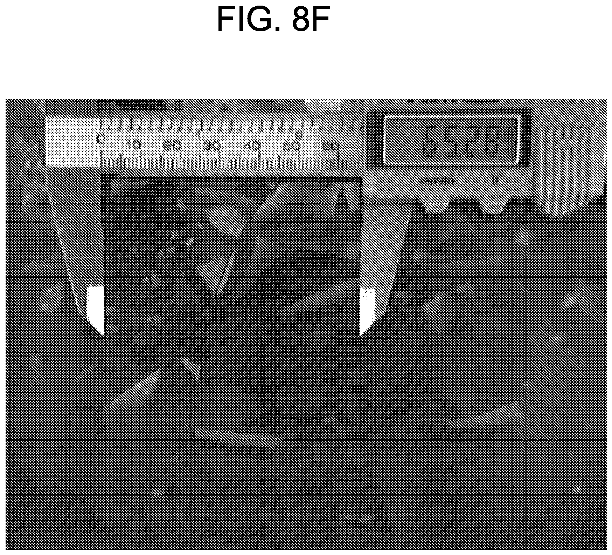

FIG. 8F illustrates an Acacia Senegal film having a flake pattern according to a reference example of the invention;

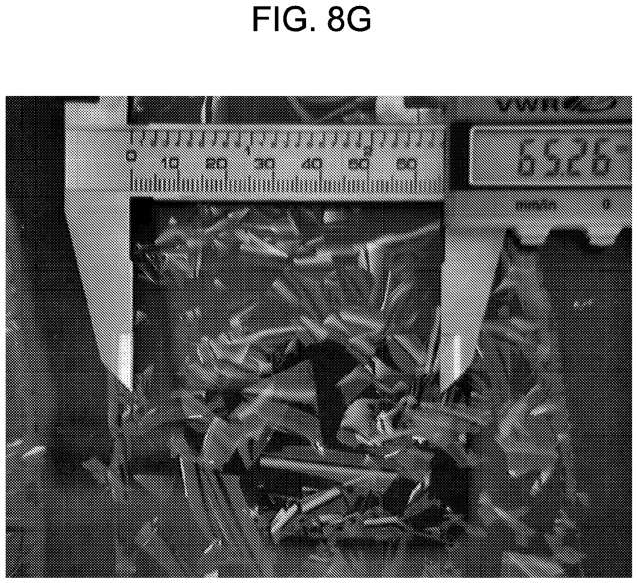

FIG. 8G illustrates an Acacia Senegal film having a flake pattern according to a reference example of the invention;

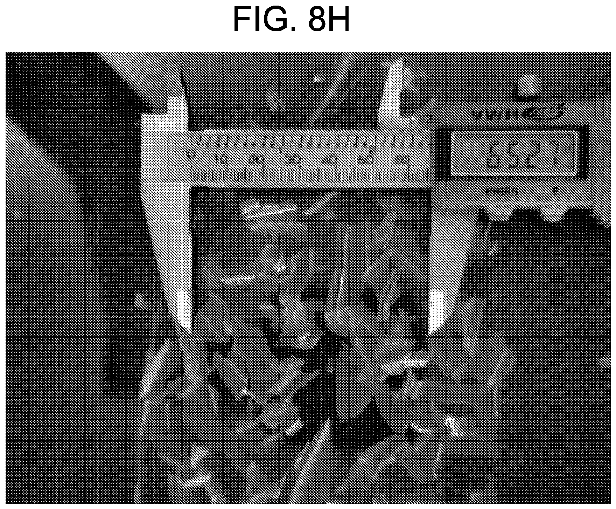

FIG. 8H illustrates a Replacement 1 Fast film having a flake pattern according to another example of the invention;

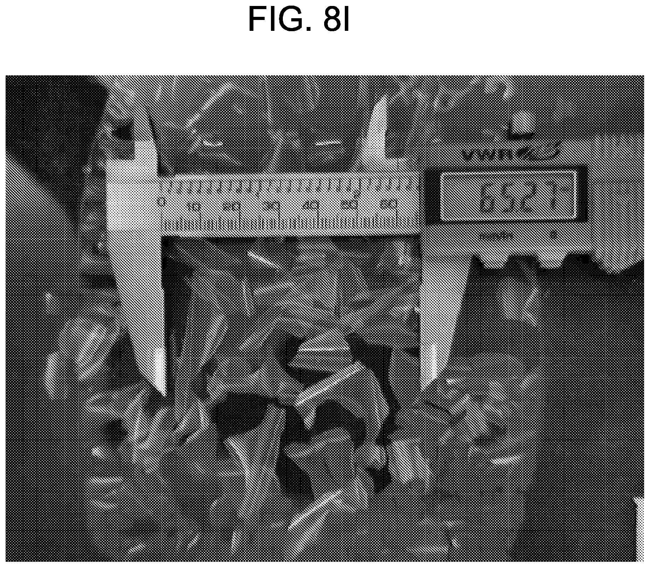

FIG. 8I illustrates a Replacement 1 All Natural film having a flake pattern according to another example of the invention;

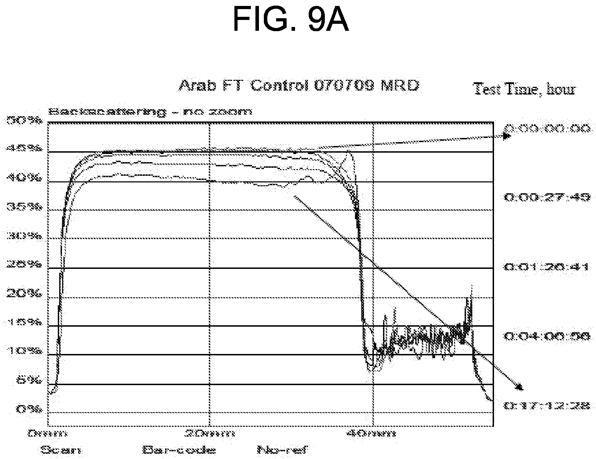

FIG. 9A illustrates a turbiscan profile of an Acacia Seyal gum arabic at different intervals of time according to a reference example of the invention;

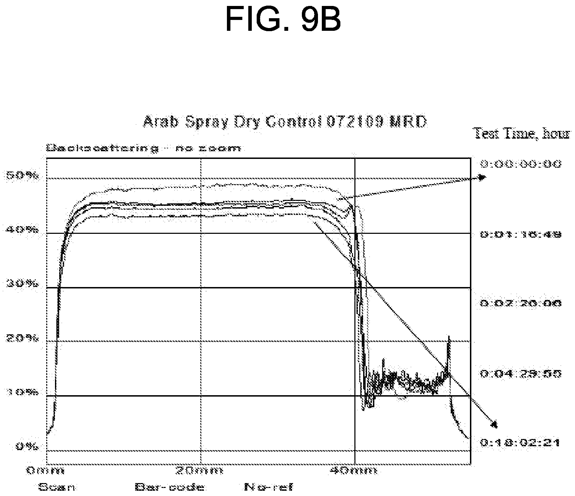

FIG. 9B illustrates a turbiscan profile of an Acacia Senegal gum Arabic at different intervals of time according to a reference example of the invention;

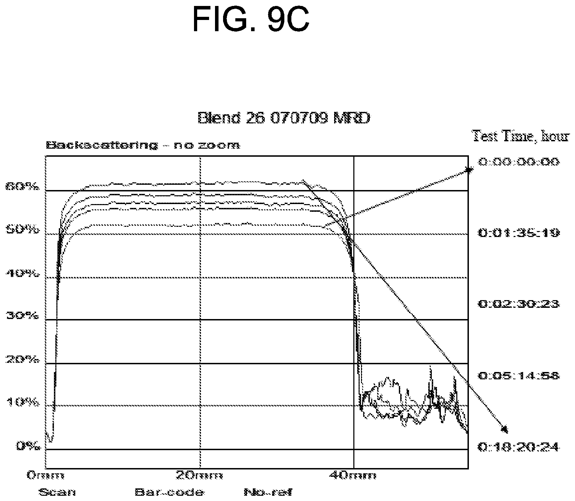

FIG. 9C illustrates a turbiscan profile of a Replacement 2 with OSA-modified gum as emulsifier at different intervals of time according to a reference example of the invention;

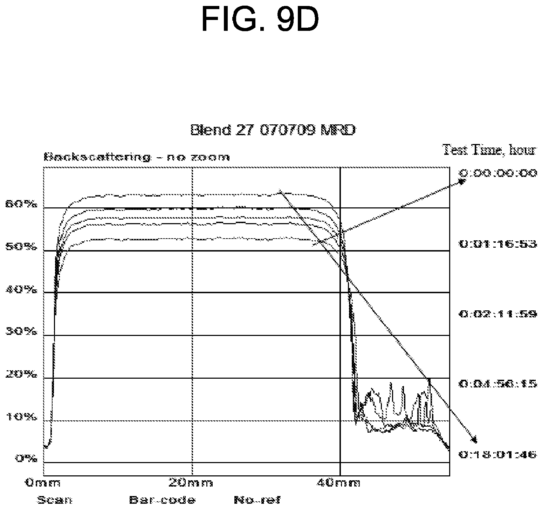

FIG. 9D illustrates a turbiscan profile of a Replacement 2 with OSA-starch emulsifier at different intervals of time according to a reference example of the invention;

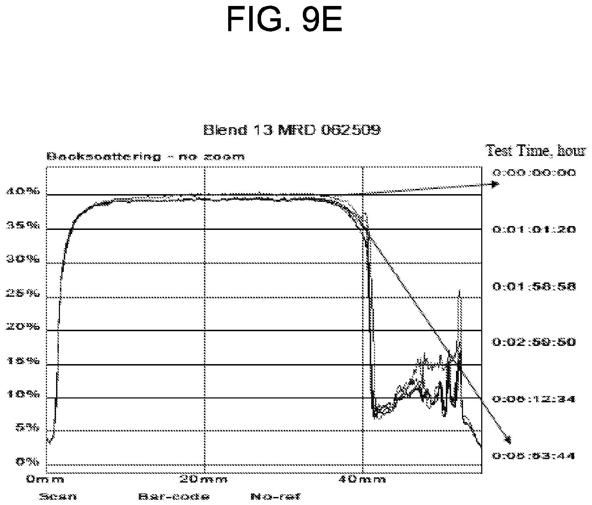

FIG. 9E illustrates a turbiscan profile of a Replacement 2 with natural emulsifier at different intervals of time according to a reference example of the invention;

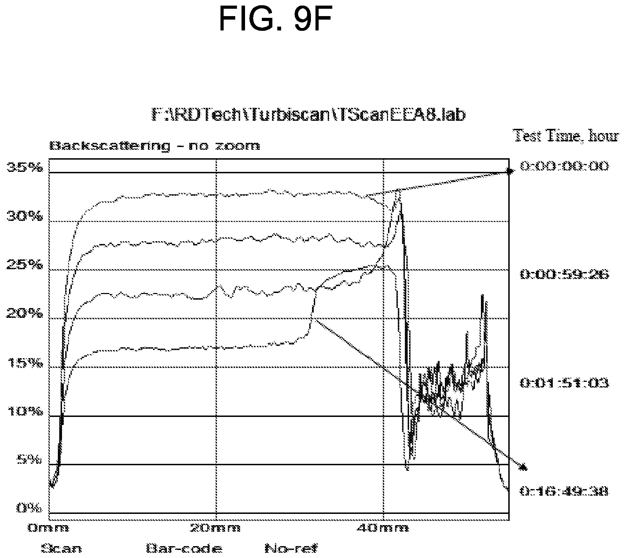

FIG. 9F illustrates a turbiscan profile of a separated emulsion-Replacement 2 without emulsifier at different intervals of time according to a reference example of the invention;

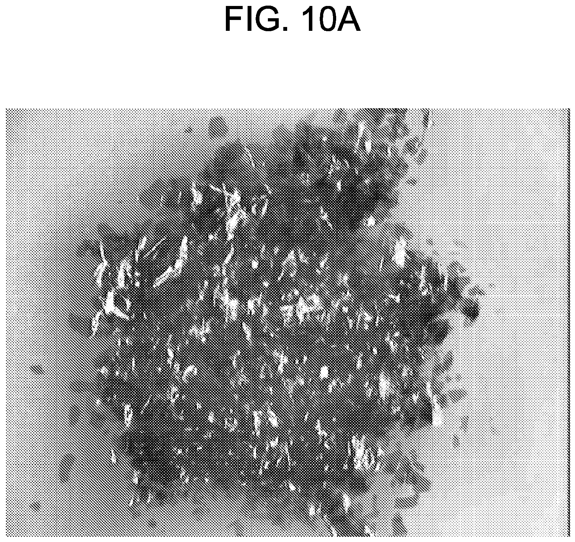

FIG. 10A illustrates an Acacia Seyal edible glitter according to a reference example of the invention;

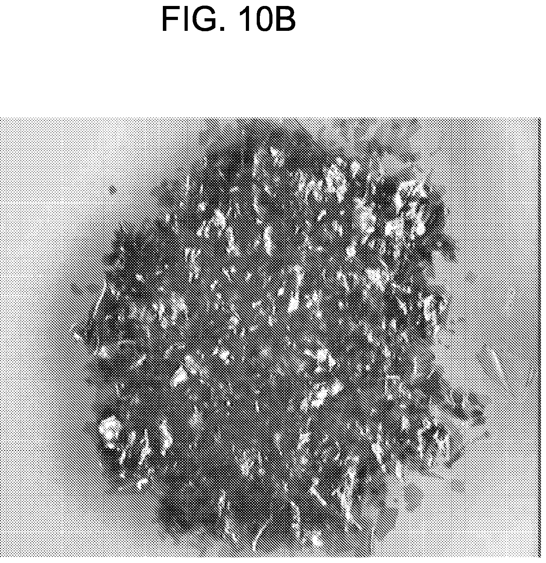

FIG. 10B illustrates a Replacement 2 with OSA-modified gum edible glitter according to a reference example of the invention; and

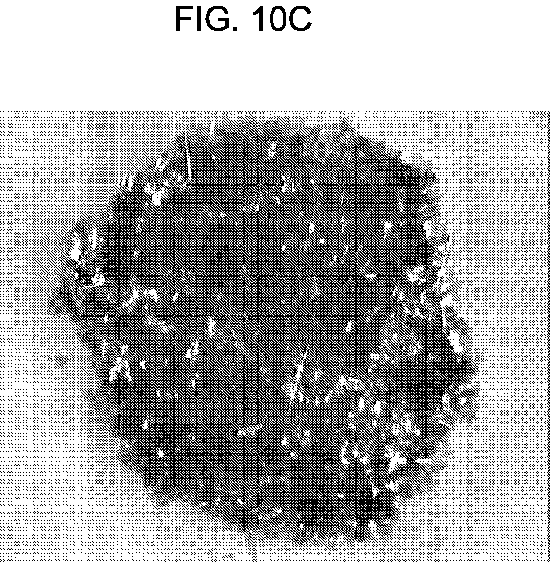

FIG. 10C illustrates Replacement 2 All Natural emulsifier edible glitter according to a reference example of the invention.

### DETAILED DESCRIPTION OF THE ILLUSTRATED EMBODIMENTS

Embodiments of the invention are directed towards the use of gum arabic replacements for panning, confections, food adhesion. Of course gum arabic replacement, partial replacement or extending composition may be used for a number of other applications, e.g., food, pharmaceutical and industrial applications. Embodiments are also directed towards replacements for gum arabic that use locally available materials for major and minor users such as the chewing gum, confection, , thereby minimizing cost and shortage concerns.

The nature of gum arabic lies between that of materials that can generate continuous films and materials that cannot generate continuous films. Embodiments of the invention, through the manipulation of key components such as strong film formers, cracking agents, fast crystallizing agents and tackiness modifier, permit formation of materials that lie anywhere between these two film forming extremes.

Embodiments of the invention also consider parameters or characteristics of gum arabic, such as film forming capability, low viscosity, sugar binding properties, effect on drying times, crystallization patterns, level of tackiness, and crunch. While these parameters or characteristics of gum arabic provide good performance in end-use applications, improvements in specific characteristics such as drying time, shell strength, crunch, and optimized tackiness are also desired. Moreover, issues concerning cracking during transportation of end products create a demand for an anti-cracking or anti-chipping gum arabic replacement.

The choice for the use of gum arabic for sealing nuts, chocolate, maltballs and other oil containing centers intended for panning also stems from its uniquely low viscosity that makes it possible to apply it as a coating syrup at 40% and from its film forming property that acts both as an oil barrier to seal oil and as an oxygen barrier to prevent oxidation and rancidity. Gum arabic is also tacky and adhesive at high concentrations, which makes it suitable for adhesion of seeds, cereal clusters, granola bars and related products. It leaves a shiny film when used as a polish for chocolate panned confections, when coated on tablets or when sprayed over chocolate bars and other candies. Gum arabic is also used in pastilles and other hard candies to increase strength and reduce the brittleness of crystallized sugar candies.

The cracking pattern of gum arabic when its solution of about 40% is cast and dried on a surface (normally a stainless steel belt) is perfect for its use in edible film glitter. Again the weak film and the low viscosity are critical properties; additionally, the clarity, sheen, adhesion property when wet and ease of release when the film dries are equally important quality attributes of gum arabic in this application.

Embodiments of the invention are directed towards preparing a gum arabic replacement, partial replacement or extending composition, for use in variety of different applications. The gum arabic replacement, partial replacement or extending composition follows a two phase protocol. The two phase protocol includes Phase 1 and Phase 2-Phase 1 is directed towards studying gum arabic properties such as viscosity, film forming characteristics, coating, sheen, crystallization, cracking and solubility. Phase 2 is directed towards testing of gum arabic replacement, partial replacement or extending composition in specific end uses, such as food, pharmaceutical and industrial applications.

Referring now to Phase 1, basic gum arabic studies of viscosity, film forming, coating, sheen, crystallization, cracking and solubility in water and high Brix syrups were conducted. Various concentrations of gum arabic, both Acacia Seyal and Acacia Senegal, between about 10 - 50% were prepared and viscosity measured.

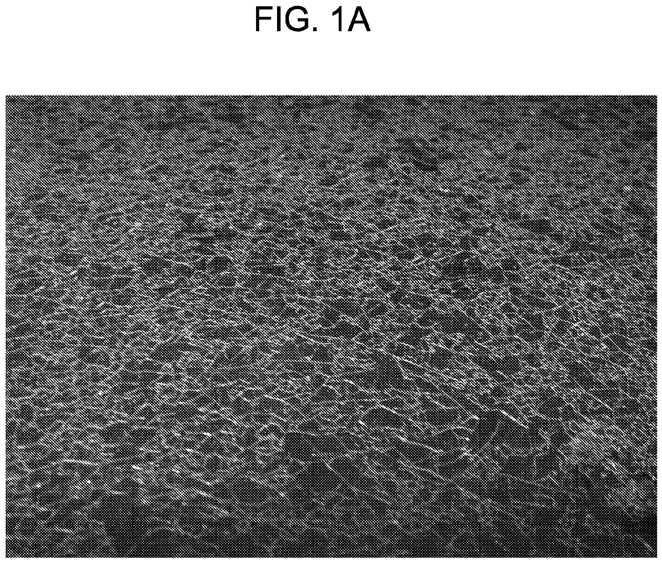

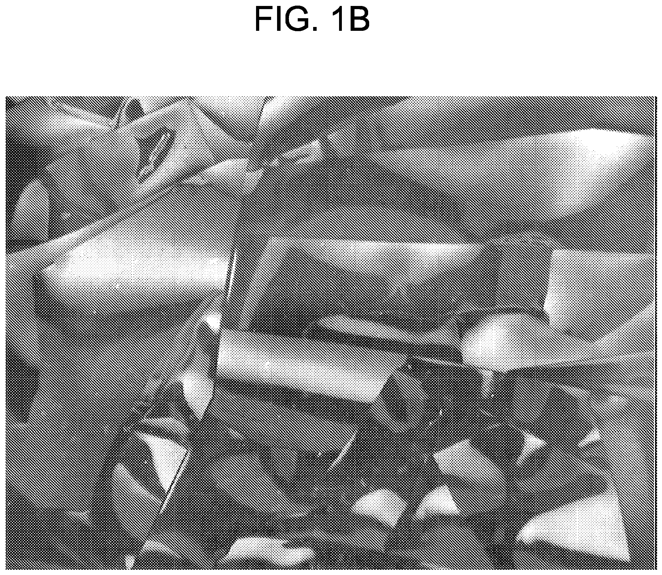

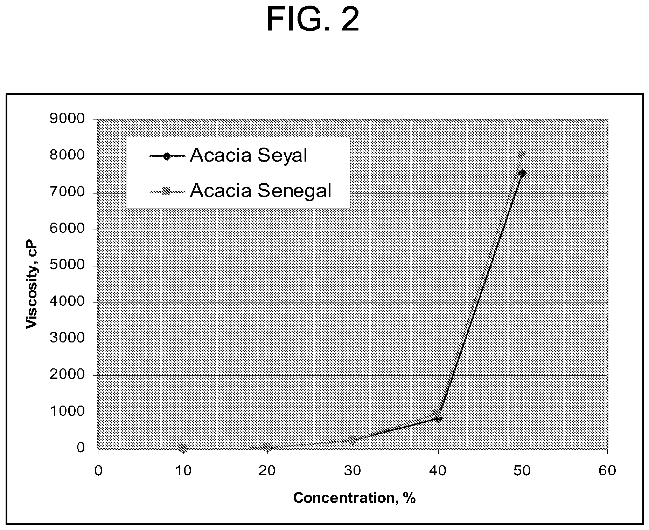

FIG. 1A illustrates a film cracking pattern of Acacia Seyal gum arabic. FIG. 1B illustrates a film cracking pattern of Acacia Senegal gum Arabic. FIG. 2 illustrates a viscosity profile of the gum arabic compositions in FIGS 1A and 1B.

Referring to FIG. 1A, a cracking pattern was formed with an Acacia Seyal gum arabic water-in-water (w/w) emulsion. The composition was about a 35 % w/w, i.e., Acacia Seyal gum arabic syrup. This composition was cast at 10 mils thickness on a glass plate between about 10 to 40 mils in thickness, dried, and observed for crystallization and cracking patterns. The resulting film formed a cracking pattern shown in FIG. 1A.

Referring to FIG. 1B, a cracking pattern was formed with an Acacia Senegal gum arabic water-in-water (w/w) emulsion. The composition was about a 35 % w/w, i.e., Acacia Senegal gum arabic syrup. This composition was cast at 10 mils thickness on a glass plate between about 10 to 40 mils in thickness, dried, and observed for crystallization and cracking. The resulting film formed a cracking pattern shown in FIG. 1B.

Comparing FIGS. 1A and 1B, there is a significant difference between the patterns, such as a difference in the number of cracks and size of flakes between the Seyal and the Senegal, the former producing numerous cracks and small flakes and the latter producing fewer cracks and larger size flakes.

FIG. 2 illustrates a viscosity profile of gum arabic compositions in FIGS. 1A and 1B. Referring to FIG. 2, it illustrates a hydration and viscosity of gum arabic in water at different concentrations, the profiles for Seyal and Senegal closely match each other. Individual gums were also studied the same way for film forming or cracking, changing concentrations as needed depending on viscosity. From the results of this study, it was determined that a single component gum arabic replacement was not able to match the attributes of gum arabic in these evaluations.

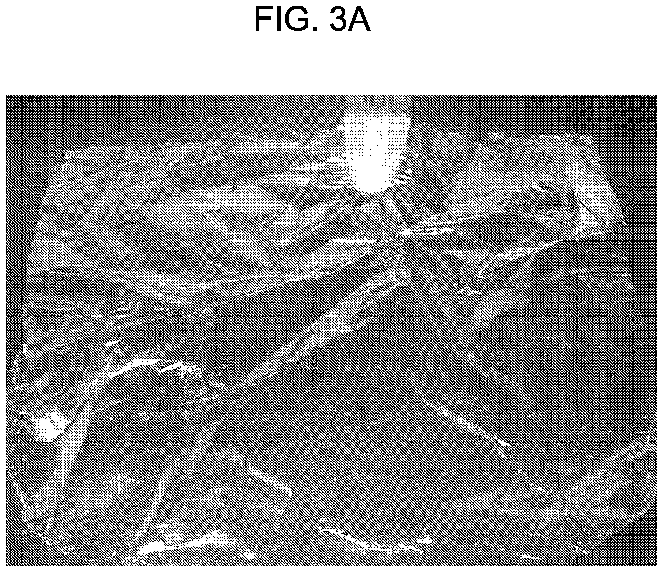

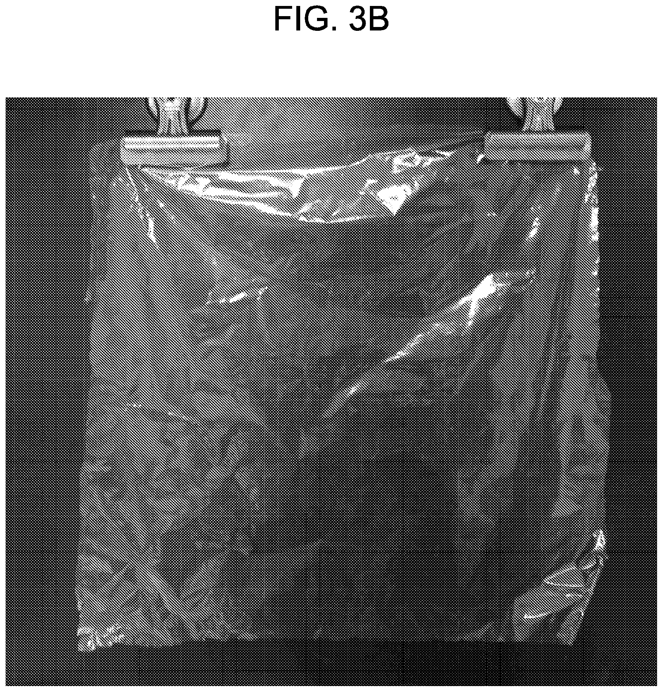

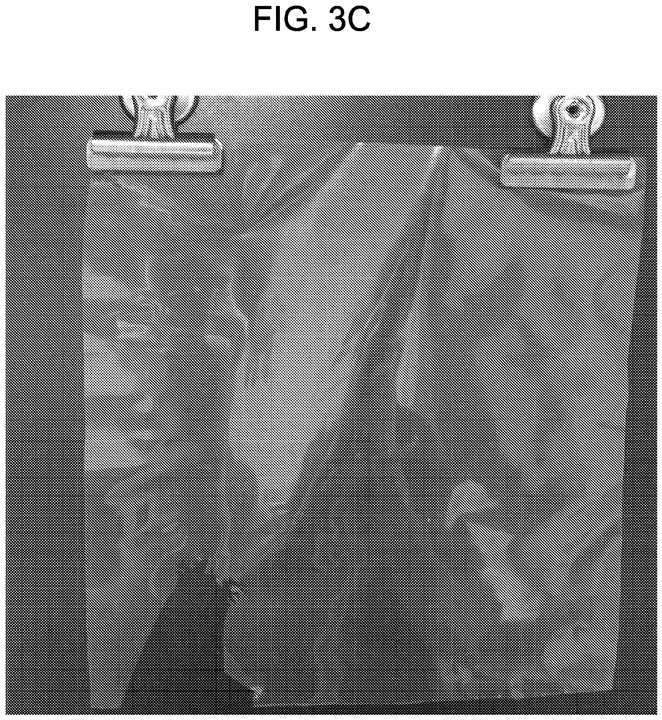

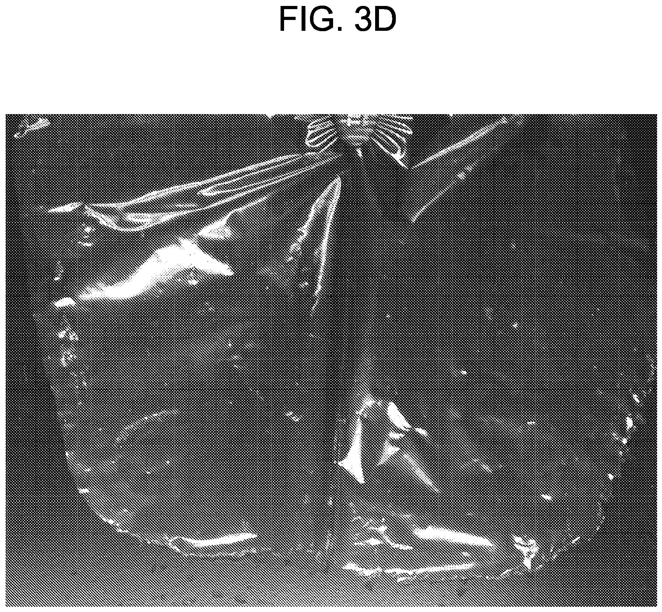

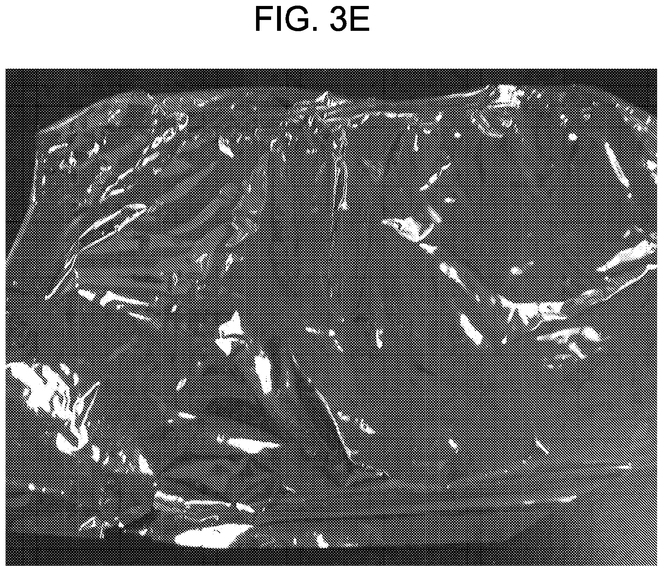

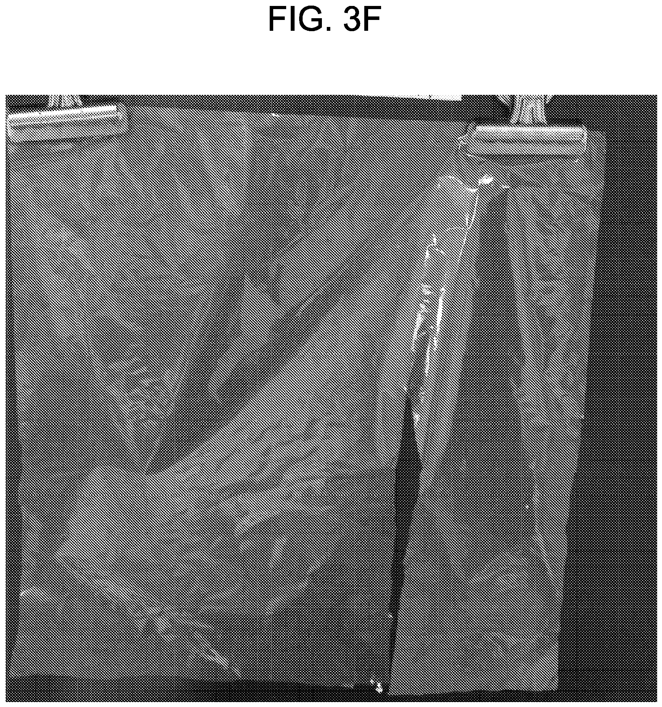

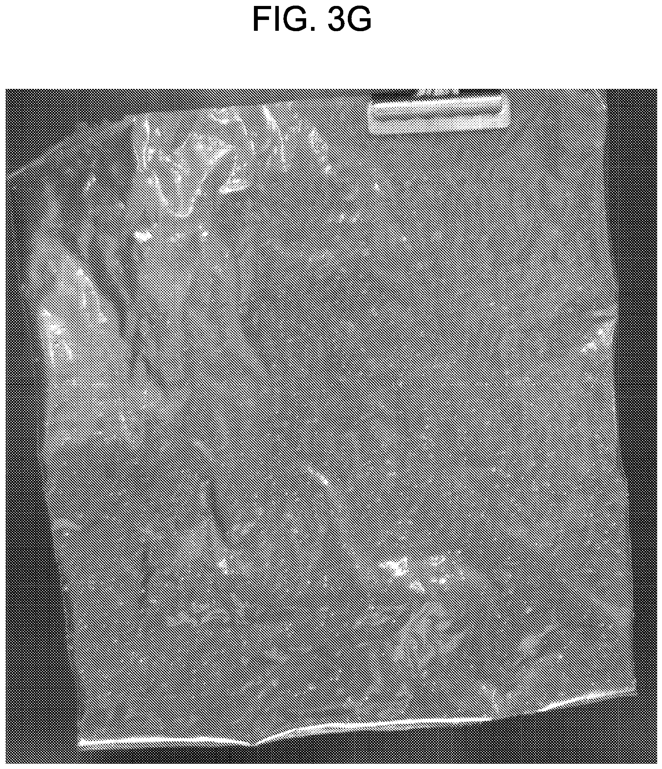

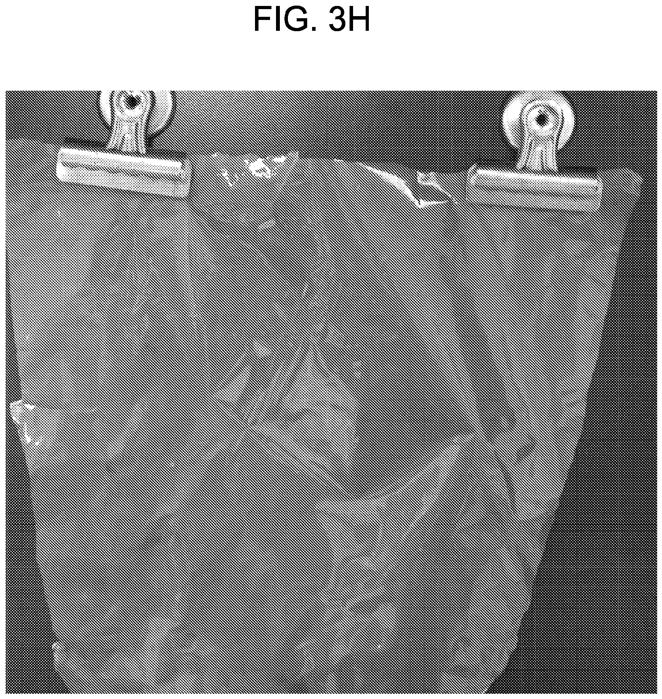

FIG. 3A illustrates a CMC or cellulose gum film according to another example of the invention. FIG. 3B illustrates a methylcellulose gum film according to another example of the invention. FIG. 3C illustrates a pectin film according to another example of the invention. FIG. 3D illustrates an agar film according to another example of the invention. FIG. 3E illustrates a sodium alginate film according to a reference example of the invention. FIG. 3F illustrates a locust bean gum film according to another example of the invention. FIG. 3G illustrates a xanthan gum film according to another example of the invention. FIG. 3H illustrates a carrageenan film according to another example of the invention.

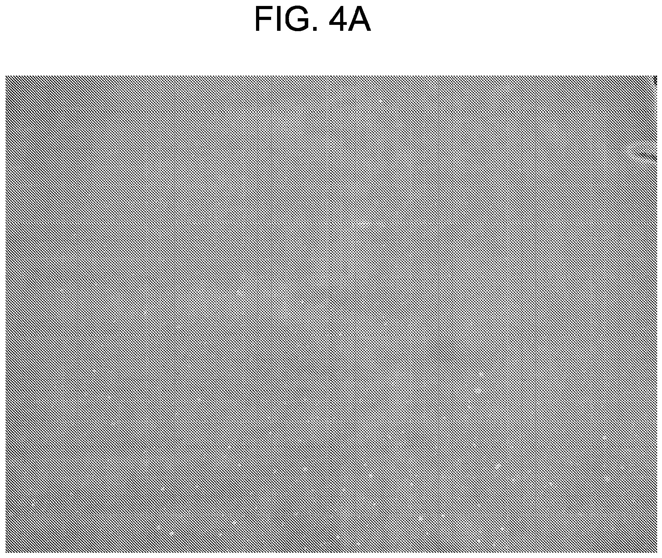

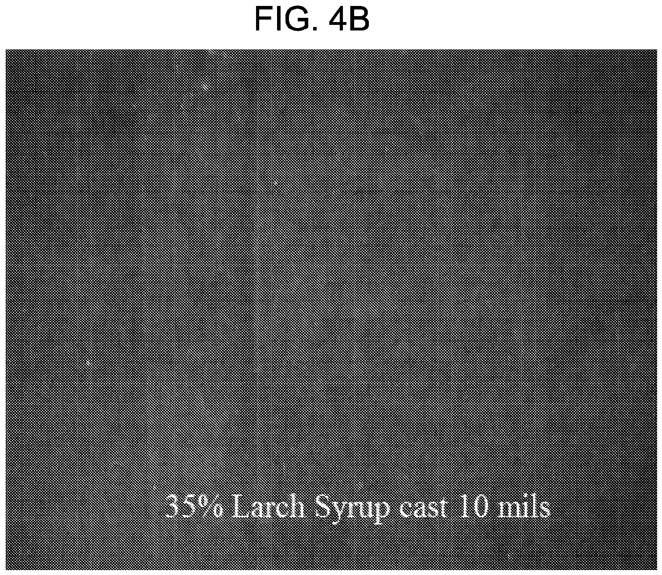

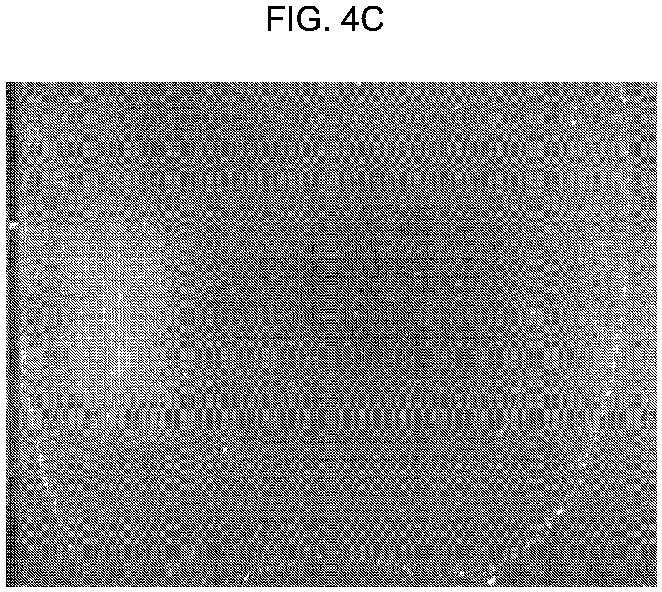

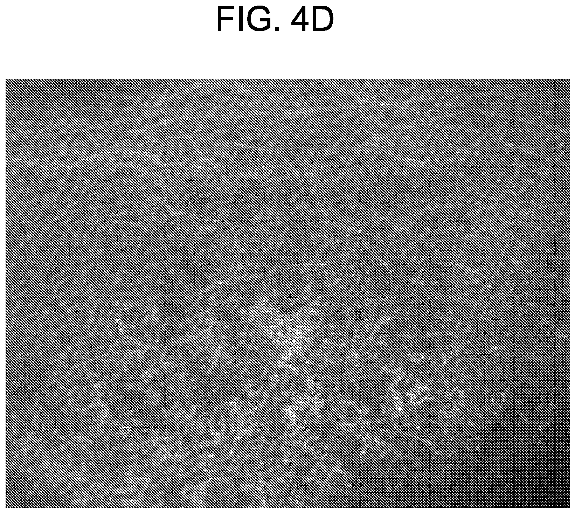

FIG. 4A illustrates an inulin film according to another example of the invention. FIG. 4B illustrates a larch gum film according to another example of the invention. FIG. 4C illustrates a low viscosity starch film according to another example of the invention. FIG. 4D illustrates a maltodextrin film according to another example of the invention.

Now referring to FIGS. 3A-4D, various properties and features can be illustrated. That is, FIGS. 3A-3H are a representation of the film forming properties of food grade gum polysaccharides prepared at suitable concentrations for casting including that of a maltodextrin film and low viscosity starch film as shown in FIGS. 4C and 4D, respectively.

From these results, it was discovered that no other material alone will replace gum arabic and that a replacement (or replacements) depending on application, can only be achieved with a carefully designed composition. Also, it is shown that the wide range of polysaccharides studied that do not form a film and turned powdery when scraped, as in the case of inulin film (FIG. 4A) and 15 DE maltodextrin film (FIG. 4D), to those that form a strong cohesive film that peels off in one piece after casting and drying, such as cellulose gum film (FIG. 3A), methylcellulose film (FIG. 3B), pectin film (FIG. 3C), agar film (FIG. 3D), sodium alginate film (FIG. 3E), locust bean gum film (FIG. 3F), xanthan film (FIG. 3G) and carrageenan film (FIG. 3H) among others. It was discovered that required elements to replace gum arabic, either Acacia Seyal and Acacia Senegal, in various applications include: (1) a strong (key) film former, that is required in all applications at some minimum level, the addition of (2) a low viscosity cracking ingredient,(3) a tackiness modifier, and (4) a fast-crystallizing ingredient; each may be required dependent on the finished application.

From a number of compositions studied with competitive or better cost structure than gum arabic, the end result is three methods of replacement that produce replacement compositions that are functionally equivalent to gum arabic in three major categories of applications. These are: (1) Replacement Method 1 is targeted for use insugar and sugar-free panning, confections, and food adhesion applications; (2) Replacements Method 2 is a gum arabic replacement more suited for flavor encapsulation/spray dried flavors and edible film and glitters applications, although they will also work in panning and confections applications.

The Method according to the invention generates replacement compositions:
1. blending a strong (key) film former chosen from a list of natural and modified polysaccharides including, but not limited to, carrageenan, pectin, alginate, gellan, agar, konjac, xanthan, guar, tara, fenugreek, cellulose gum, methyl cellulose, HPMC, propylene glycol alginate, or a combination thereof at approximately 0.1 % to 10% when replacing gum arabic or used at a level in the finished application equivalent to about 0.1 to 10% of what gum arabic would typically be used (regardless of whether gum arabic is used), where such key film formers may vary in molecular weights and viscosities;
2. a low viscosity cracking ingredient that standardizes the viscosity, modifies the crystallization and cracking pattern of the key film former in 1 above including, but not limited to, maltodextrin, monosaccharides, disaccharides, oligosaccharides, larch gum, polydextrose, or a combination thereof, that is chosen depending on if the application is sugar-free or not and where such ingredient, or combination thereof, may be used at concentrations from 0% to 99.9%;
3. adding a fast-crystallizing ingredient that may also serve the role as a cracking agent consisting of, but not limited to, inulin, erythritol, low DE maltodextrin, lactose, or a combination thereof, used at 0% to 99.9%; and
4. adding a tackiness modifier consisting of, but not limited to, starch, guar gum, locust bean gum, or a combination thereof, at approximately 0% to 10%, where such ingredient may vary in molecular weights and viscosities.

A Method 2 generates comparative replacement compositions:
1. selecting a strong (key) film former chosen from a list of natural and modified polysaccharides including, but not limited to, carrageenan, pectin, alginate, gellan, agar, konjac, xanthan, guar, locust bean gum, tara, fenugreek, starch, cellulose gum, methyl cellulose, HPMC, propylene glycol alginate, or a combination thereof at approximately 0.1 to 10% when replacing gum arabic or used at a level in the finished application equivalent to about 0.1 to 10% of what gum arabic would typically be used (regardless of whether gum arabic is used), where such key film former may vary in molecular weights and viscosities;
2. adding an emulsifier such as propylene glycol alginate, emulsifying starch, OSA-modified gums, mono- and diglycerides, soy lecithin, proteins or a combination thereof, at approximately 0.1% to 25%; and
3. adding a low viscosity polysaccharide such as maltodextrin, larch gum, polydextrose, inulin, or a combination thereof, used at concentrations from 50% to 99.9%, and to achieve a drop-in usage as gum arabic or a reduction in usage in some non-sensitive applications with equivalent functionality as gum arabic.

Phase 2 involved testing of the gum arabic replacement compositions in specific end uses, such as: (1) The Method replacements compositions according to the invention referred to as Replacement 1 Std. as a match to Acacia Seyal, Replacement 1 Fast and Replacement 1 all natural were used in sugar-free panning of chewing gums, sealing and regular sugar panning of peanuts, as well as in pastilles; (2) Reference Method 2 replacement compositions labeled Replacements 2 were tested in flavor encapsulation and film glitter applications.

Table 1 was prepared and includes size of flakes for a 40°Brix Acacia Seyal, Acacia Senegal, Seyal-Senegal blend and gum Replacements from Method according to the invention cast at 5 and 10 mils thickness.

**TABLE 1:**

| Gum System | Width, mm | Length, mm |
|---|---|---|
| | | |
| Acacia Seyal, 100% | 0.5 - 3 | 1.0 - 10 |
| Acacia Seyal/Senegal, 50:50 | 3 - 10 | 4 - 30 |
| Acacia Senegal, 100% | 5 - 23 | 10 - 60 |
| Replacement 1 | 0.5 - 3 | 1.0 - 10 |
| Replacement 1 Fast | 5 - 30 | 10 - 70 |
| Replacement 1 Fast All Natural | 3 - 20 | 10 - 40 |

**Sugar-Free Chewing Gum Panning Example:** In this example, gum arabic was compared to Replacement 1 Std. and Replacement 1 Fast. The sugar-free syrup recipe included 64.5% maltitol, 3.5% binder (gum arabic control or the gum arabic Replacements) and 32% water. The gums were first added to the water and heated to about 82°C, before the maltitol powder was added. Then the syrups were brought to a boil, adjusted to 70°Brix, 68°Brix and 60°Brix for viscosity measurements at 60°C and 25°C. Using an 18 inch diameter pan coater, the 60°Brix syrups containing gum arabic or the replacements were used in panning and compared for stickiness/tackiness and drying characteristics. The finished chewing gums with about 32.4 to 32.6% shell coat were equilibrated in the humidity chamber for about 16 hours at 35°C and 12% RH and were tested for shell strength using the TA XT Plus Texture Analyzer, needle probe, 1 mm/sec test speed on 50 pieces of chewing gums as replications.

Viscosities of the sugar-free syrups are all in line with each other for gum arabic and the replacements as shown in Table 2. Table 2 illustrates syrup viscosities of maltitol syrups containing gum arabic and the replacements at application temperatures of 25°C and 60°C and various Brix values. Also, it was found that higher syrup viscosity may contribute to the centers sticking together and a bumpy surface and are, hence, undesirable. The different Replacement compositions were, therefore, all standardized to give the same viscosity and tackiness as the gum arabic, except in Replacement 1 Fast where tackiness is reduced for ease of panning.

**TABLE 2:**

| Gum System | Average Viscosity at 25°C, cP | | | Average Viscosity at 60°C, cP | | |
|---|---|---|---|---|---|---|
| | 70°Brix | 68°Brix | 60°Brix | 70°Brix | 68°Brix | 60°Brix |
| Acacia Seyal | 511 | 340 | 87 | 73 | 59 | 27 |
| Repl 1 Std. | 531 | 374 | 98 | 77 | 59 | 28 |
| Repl 1 Fast | 514 | 460 | 96 | 89 | 70 | 29 |
| Repl 1 All Natural | 460 | 350 | 92.5 | 60 | 54 | 29 |

Table 3 includes experimental batch records of sugar-free chewing gum panning using an 18 inch pan coater (batch size of 1000 g; dusting powder is maltitol; syrup temperature at a temperature of 60°C; center size is 1.0 cm x 2.1 cm; drying time after free flow was 2.5 minutes). Table 4 includes experimental batch records of sugar-free chewing gum panning using an 18 inch pan coater (batch size of 700 g; dusting powder is maltitol; center size is 1.2 cm x 1.9 cm; drying time after free flow is 2.5 minutes). As shown in Tables 3 and 4, Replacement 1 Std. shows the same drying time as the gum arabic. Replacement 1 Fast, which contains a fast-crystallizing ingredient, showed faster drying per syrup charge demonstrating that, as needed, the crystallization and drying of the panning syrup based on the standard replacement composition can be manipulated and improved over that of gum arabic. With a process as time consuming as panning, shorter drying per charge is desirable which is estimated to be in the magnitude of between 5-10% faster using an open pan coater. At the same time, an improvement in the color is achieved using the replacements that were both whiter in color. Gum arabic has a natural brownish pigment that shows in the shell coating especially if the chewing gums are panned without color.

**TABLE 3:**

| Charge # | | | | Maltitol-Gum Arabic Syrup | | Maltitol-Repl 1 Std Syrup | | Maltitol-Repl1 Fast Syrup | |
|---|---|---|---|---|---|---|---|---|---|
| | Syrup wt, g | Mix Time After Syrup | Mix Time Dusting Powder | Air to Free Flow, min | Cycle Time, min | Free Flow, min | Cycle Time, min | Air to Free Flow, min | Cycle Time, min |
| Precoat/Gumming | | | | | | | | | |
| 1 | 12.5 | 1 | 1 | x | 5.50 | x | 5.50 | x | 5.50 |
| 2 | 12.5 | 1 | 1 | x | 5.50 | x | 5.50 | x | 5.50 |
| 3 | 12.5 | 1 | 1 | x | 5.50 | x | 5.50 | x | 5.50 |
| 4 | 12.5 | 1 | 1 | x | 5.50 | x | 5.50 | x | 5.50 |
| 5 | 12.5 | 1 | 1 | x | 5.50 | x | 5.50 | x | 5.50 |

| Engrossing [Same conditions up to 25 charges of syrup] | | | | | | | | | |
|---|---|---|---|---|---|---|---|---|---|
| 6 | 6.25 | 1 | x | 2.5 | 5.00 | 2.5 | 5.00 | 2.5 | 5.00 |
| 7 | 6.25 | 1 | x | 2.5 | 5.00 | 2.5 | 5.00 | 2.5 | 5.00 |
| 8 | 6.25 | 1 | x | 2.5 | 5.00 | 2.5 | 5.00 | 2.5 | 5.00 |
| 9 | 6.25 | 1 | x | 2.5 | 5.00 | 2.5 | 5.00 | 2.5 | 5.00 |
| 10 | 6.25 | 1 | x | 2.5 | 5.00 | 2.5 | 5.00 | 2.5 | 5.00 |
| 11 | 6.25 | 1 | x | 3.50 | 6.00 | 3.50 | 6.00 | 3.50 | 6.00 |
| 12 | 6.25 | 1 | x | 3.50 | 6.00 | 3.50 | 6.00 | 3.50 | 6.00 |
| 13 | 6.25 | 1 | x | 3.50 | 6.00 | 3.50 | 6.00 | 3.50 | 6.00 |
| 14 | 6.25 | 1 | x | 3.50 | 6.00 | 3.50 | 6.00 | 3.50 | 6.00 |
| 15 | 6.25 | 1 | x | 3.50 | 6.00 | 3.50 | 6.00 | 3.50 | 6.00 |
| 16 | 6.25 | 1 | x | 3.50 | 6.00 | 3.50 | 6.00 | 3.50 | 6.00 |
| 17 | 6.25 | 1 | x | 3.50 | 6.00 | 3.50 | 6.00 | 3.50 | 6.00 |
| 18 | 6.25 | 1 | x | 3.50 | 6.00 | 3.50 | 6.00 | 3.50 | 6.00 |
| 19 | 6.25 | 1 | x | 3.50 | 6.00 | 3.50 | 6.00 | 3.50 | 6.00 |
| 20 | 6.25 | 1 | x | 3.50 | 6.00 | 3.50 | 6.00 | 3.50 | 6.00 |
| 21 | 6.25 | 1 | x | 3.50 | 6.00 | 3.50 | 6.00 | 3.50 | 6.00 |
| 22 | 6.25 | 1 | x | 3.50 | 6.00 | 3.50 | 6.00 | 3.50 | 6.00 |
| 23 | 6.25 | 1 | x | 3.50 | 6.00 | 3.50 | 6.00 | 3.50 | 6.00 |
| 24 | 6.25 | 1 | x | 3.50 | 6.00 | 3.50 | 6.00 | 3.50 | 6.00 |
| 25 | 6.25 | 1 | x | 3.50 | 6.00 | 3.50 | 6.00 | 3.50 | 6.00 |
| | | | | Non-sticky on charge 19 | | Non-sticky on charge 16 | | Non-sticky on charge 16 | |
| 26 | 9.37 | 1 | x | 3.00 | 5.50 | 2.83 | 5.33 | 2.25 | 4.92 |
| 27 | 9.37 | 1 | x | 2.57 | 5.07 | 2.57 | 5.07 | 2.50 | 5.00 |
| 28 | 9.37 | 1 | x | 2.50 | 5.00 | 2.50 | 5.00 | 2.57 | 5.07 |
| 29 | 9.37 | 1 | x | 2.33 | 4.83 | 2.35 | 4.85 | 2.35 | 4.85 |
| 30 | 9.37 | 1 | x | 2.33 | 4.83 | 2.37 | 4.87 | 2.37 | 4.87 |
| 31 | 9.37 | 1 | x | 2.17 | 4.67 | 2.17 | 4.67 | 2.08 | 4.58 |
| 32 | 9.37 | 1 | x | 2.25 | 4.75 | 2.33 | 4.83 | 2.25 | 4.75 |
| 33 | 9.37 | 1 | x | 2.50 | 5.00 | 2.50 | 5.00 | 2.17 | 4.67 |
| 34 | 9.37 | 1 | x | 2.50 | 5.00 | 2.50 | 5.00 | 2.17 | 4.40 |
| 35 | 9.37 | 1 | x | 2.42 | 4.92 | 2.67 | 5.16 | 2.08 | 4.58 |
| 36 | 9.37 | 1 | x | 2.25 | 4.75 | 2.25 | 4.75 | 2.00 | 4.50 |
| 37 | 9.37 | 1 | x | 2.08 | 4.58 | 2.08 | 4.58 | 2.08 | 4.58 |
| 38 | 9.37 | 1 | x | 2.08 | 4.58 | 2.08 | 4.58 | 2.08 | 4.58 |
| 39 | 9.37 | 1 | x | 2.08 | 4.58 | 2.17 | 4.67 | 2.08 | 4.58 |
| 40 | 9.37 | 1 | x | 2.08 | 4.58 | 2.17 | 4.67 | 2.00 | 4.50 |
| 41 | 9.37 | 1 | x | 2.33 | 4.83 | 2.33 | 4.83 | 2.00 | 4.50 |
| 42 | 9.37 | 1 | x | 2.17 | 4.67 | 2.33 | 4.83 | 2.08 | 4.58 |
| 43 | 9.37 | 1 | x | 2.17 | 4.67 | 2.42 | 4.92 | 2.08 | 4.58 |
| 44 | 9.37 | 1 | x | 2.33 | 4.83 | 2.33 | 4.83 | 2.08 | 4.58 |
| 45 | 9.37 | 1 | x | 2.33 | 4.83 | 2.42 | 4.92 | 2.08 | 4.58 |
| 46 | 9.37 | 0 | x | 3.67 | 6.17 | 3.92 | 6.33 | 3.33 | 5.83 |
| 47 | 9.37 | 0 | x | 3.75 | 6.25 | 3.75 | 6.25 | 3.00 | 5.50 |
| 48 | 9.37 | 0 | x | 3.67 | 6.17 | 3.58 | 6.08 | 2.83 | 5.33 |
| 49 | 9.37 | 0 | x | 3.67 | 6.17 | 3.67 | 6.17 | 3.00 | 5.50 |
| 50 | 9.37 | 0 | x | 3.92 | 6.42 | 3.67 | 6.17 | 2.75 | 5.25 |
| 51 | 9.37 | 0 | x | 4.00 | 6.50 | 3.83 | 6.33 | 3.33 | 5.83 |
| 52 | 9.37 | 0 | x | 4.17 | 6.67 | 4.00 | 6.50 | 3.00 | 5.50 |
| 53 | 9.37 | 0 | x | 4.00 | 6.50 | 4.00 | 6.50 | 3.00 | 5.50 |
| 54 | 9.37 | 0 | x | 3.67 | 6.17 | 3.58 | 6.08 | 2.92 | 5.54 |
| 55 | 9.37 | 0 | x | 3.92 | 6.63 | 3.75 | 6.25 | 3.00 | 5.50 |
| 56 | 12.5 | 0 | x | 4.00 | 6.50 | 3.83 | 6.33 | 2.92 | 5.42 |
| 57 | 12.5 | 0 | x | 4.17 | 6.67 | 4.29 | 6.50 | 2.92 | 5.42 |
| 58 | 12.5 | 0 | x | 4.00 | 6.50 | 4.00 | 6.50 | 3.00 | 5.50 |
| 59 | 12.5 | 0 | x | 3.67 | 6.17 | 3.58 | 6.08 | 3.00 | 5.50 |
| 60 | 12.5 | 0 | x | 3.92 | 6.42 | 3.75 | 6.25 | 3.00 | 5.50 |
| 61 | 12.5 | 0 | x | 3.17 | 5.67 | 3.25 | 5.75 | 2.67 | 5.17 |
| 62 | 12.5 | 0 | x | 3.08 | 5.58 | 3.08 | 5.58 | 3.00 | 5.50 |
| 63 | 12.5 | 0 | x | 3.25 | 5.75 | 3.33 | 5.83 | 2.50 | 5.00 |
| 64 | 12.5 | 0 | x | 3.67 | 6.17 | 3.17 | 5.67 | 2.58 | 5.08 |
| 65 | 12.5 | 0 | x | 3.58 | 6.08 | 3.25 | 5.75 | 2.67 | 5.17 |
| 66 | 12.5 | 0 | x | 3.00 | 5.50 | 3.33 | 5.83 | 2.88 | 5.38 |
| 67 | 12.5 | 0 | x | 3.03 | 5.53 | 3.18 | 5.68 | 2.83 | 5.33 |
| 68 | 12.5 | 0 | x | 2.83 | 5.33 | 2.88 | 5.38 | 2.75 | 5.25 |
| 69 | 12.5 | 0 | x | 2.83 | 5.33 | 2.83 | 5.33 | 2.68 | 5.18 |
| 70 | 12.5 | 0 | x | 2.68 | 5.18 | 2.66 | 5.16 | 2.45 | 4.95 |
| Total Time, minutes [Charges 26-70] | | | | | 248.50 | | 247.64 | | 227.88 |

**TABLE 4:**

| | | | | Maltitol-Gum Arabic | | Maltitol-Repl 1 Std. | | Maltitol-Repl1 Fast | |
|---|---|---|---|---|---|---|---|---|---|
| Charge # | Syrup wt, g | Syrup Temp. °C | Mix Time After Syrup | Air to Free Flow, min | Cycle Time, min | Free Flow, min | Cycle Time, min | Air to Free Flow, min | Cycle Time, min |
| 26 | 6.1 | 60 | 1 | 2.25 | 4.75 | 2.25 | 4.75 | 2.00 | 4.50 |
| 27 | 6.1 | 60 | 1 | 2.08 | 4.58 | 2.08 | 4.58 | 2.08 | 4.58 |
| 28 | 6.1 | 60 | 1 | 2.08 | 4.58 | 2.08 | 4.58 | 2.08 | 4.58 |
| 29 | 6.1 | 60 | 1 | 2.08 | 4.58 | 2.17 | 4.67 | 2.08 | 4.58 |
| 30 | 6.1 | 60 | 1 | 2.08 | 4.58 | 2.17 | 4.67 | 2.00 | 4.50 |
| 31 | 6.1 | 60 | 1 | 2.33 | 4.84 | 2.33 | 4.67 | 2.00 | 4.50 |
| 32 | 6.1 | 60 | 1 | 2.17 | 4.67 | 2.33 | 4.83 | 2.08 | 4.58 |
| 33 | 6.1 | 60 | 1 | 2.17 | 4.67 | 2.42 | 4.83 | 2.08 | 4.58 |
| 34 | 6.1 | 60 | 1 | 2.33 | 4.83 | 2.33 | 4.83 | 2.08 | 4.58 |
| 35 | 6.1 | 60 | 1 | 2.33 | 4.83 | 2.42 | 4.92 | 2.08 | 4.58 |
| 36 | 6.1 | 25 | 1 | 3.00 | 5.50 | 2.83 | 5.33 | 2.25 | 4.92 |
| 37 | 6.1 | 25 | 1 | 2.57 | 5.07 | 2.57 | 5.07 | 2.50 | 5.00 |
| 38 | 6.1 | 25 | 1 | 2.50 | 5.00 | 2.50 | 5.00 | 2.57 | 5.07 |
| 39 | 6.1 | 25 | 1 | 2.33 | 4.83 | 2.35 | 4.85 | 2.35 | 4.85 |
| 40 | 6.1 | 25 | 1 | 2.33 | 4.83 | 2.37 | 4.87 | 2.37 | 4.87 |
| 41 | 6.1 | 25 | 1 | 2.17 | 4.67 | 2.17 | 4.67 | 2.08 | 4.58 |
| 42 | 6.1 | 25 | 1 | 2.25 | 4.75 | 2.33 | 4.83 | 2.25 | 4.75 |
| 43 | 6.1 | 25 | 1 | 2.50 | 5.00 | 2.50 | 5.00 | 2.17 | 4.67 |
| 44 | 6.1 | 25 | 1 | 2.50 | 5.00 | 2.50 | 5.00 | 2.17 | 4.40 |
| 45 | 6.1 | 25 | 1 | 2.42 | 4.92 | 2.67 | 5.16 | 2.08 | 4.58 |
| 46 | 6.1 | 60 | x | 3.67 | 6.17 | 3.92 | 6.33 | 3.33 | 5.83 |
| 47 | 6.1 | 60 | x | 3.75 | 6.25 | 3.75 | 6.25 | 3.00 | 5.50 |
| 48 | 6.1 | 60 | x | 3.67 | 6.17 | 3.58 | 6.08 | 2.83 | 5.33 |
| 49 | 6.1 | 60 | x | 3.67 | 6.17 | 3.67 | 6.17 | 3.00 | 5.50 |
| 50 | 6.1 | 60 | x | 3.92 | 6.42 | 3.67 | 6.17 | 2.75 | 5.25 |
| 51 | 6.1 | 60 | x | 4.00 | 6.50 | 3.83 | 6.33 | 3.33 | 5.83 |
| 52 | 6.1 | 60 | x | 4.17 | 6.67 | 4.00 | 6.50 | 3.00 | 5.50 |
| 53 | 6.1 | 60 | x | 4.00 | 6.50 | 4.00 | 6.50 | 3.00 | 5.50 |
| 54 | 6.1 | 60 | x | 3.67 | 6.17 | 3.58 | 6.08 | 2.92 | 5.42 |
| 55 | 6.1 | 60 | x | 3.92 | 6.63 | 3.75 | 6.25 | 3.00 | 5.50 |
| 56 | 6.1 | 25 | x | 4.00 | 6.50 | 3.83 | 6.33 | 2.92 | 5.42 |
| 57 | 6.1 | 25 | x | 4.17 | 6.67 | 4.90 | 6.50 | 2.92 | 5.42 |
| 58 | 6.1 | 25 | x | 4.00 | 6.50 | 4.00 | 6.50 | 3.00 | 5.50 |
| 59 | 6.1 | 25 | x | 3.67 | 6.17 | 3.58 | 6.08 | 3.00 | 5.50 |
| 60 | 6.1 | 25 | x | 3.92 | 6.42 | 3.75 | 6.25 | 3.00 | 5.50 |
| 61 | 6.1 | 25 | x | 3.17 | 5.67 | 3.25 | 5.75 | 2.67 | 5.17 |
| 62 | 6.1 | 25 | x | 3.08 | 5.58 | 3.08 | 5.58 | 3.00 | 5.50 |
| 63 | 6.1 | 25 | x | 3.25 | 5.75 | 3.33 | 5.83 | 2.50 | 5.00 |
| 64 | 6.1 | 25 | x | 3.67 | 6.17 | 3.17 | 5.67 | 2.58 | 5.08 |
| 65 | 6.1 | 25 | x | 3.58 | 6.08 | 3.25 | 5.75 | 2.67 | 5.17 |
| Total Time [charges 25-65], min | | | | | 221.64 | | 220.01 | | 201.67 |

Table 5 includes hardness of chewing gum sugar shell comparing gum arabic (Acacia Seyal) and Replacements 1 Std. and Replacement 1 Fast. The hardness of chewing gum sugar shell in Table 5 shows comparable or higher fracture Force values for the Replacements over gum arabic. Replacement 1 Fast, which contains a fast crystallizing ingredient, even when used at 2/3 the usage of gum arabic shows quite comparable shell strength in gum center #1. When used at the same usage in chewing gum center #2, Replacement 1 Fast yielded a significantly higher fracture Force compared to gum arabic and Replacement 1 Std, an improvement in functionality that is achieved by manipulating the base composition.

**TABLE 5:**

| | Maltitol-Gum Arabic | | Maltitol-Repl 1 Std | | Maltitol-Repl 1 Fast | |
|---|---|---|---|---|---|---|
| | | | | | | |

| | Force | Distance | Force | Distance | Force | Distance |
|---|---|---|---|---|---|---|
| | g | mm | g | Mm | g | mm |
| | | | | | | |

| Center #1 [1.0 cm x 2.1 cm] | | | | | | |
|---|---|---|---|---|---|---|
| Average: | 828.0 | 0.25 | 944.5 | 0.27 | 781.6* | 0.23 |
| Std. Dev. | 99.7 | 0.044 | 56.8 | 0.04 | 59.2 | 0.043 |
| n | 50.0 | | 50.0 | | 50 | |
| | | | | | | |

| Center #2 [1.1 cm x 1.9 cm] | | | | | | |
|---|---|---|---|---|---|---|
| Average: | 647.2 | 0.202 | 683.0 | 0.233 | 757.5 | 0.273 |
| Std. Dev. | 84.6 | 0.028 | 134.7 | 0.034 | 148.8 | 0.103 |
| n | 50.0 | | 50.0 | | 50 | |

| | | | | | | |
|---|---|---|---|---|---|---|
| *at 2/3 the usage in the syrup | | | | | | |

**Sealing of Peanuts Example:** In this example, gum arabic replacements were also tested in sealing of peanuts to provide both oil and oxygen barriers, by applying the syrup at 40°Brix in 3 charges with and without dusting powder but drying in between syrup charges. Viscosities of these syrups are shown in Table 6 illustrating viscosities of 40°Brix sealing syrup comparing gum arabic and Replacement 1 according to the invention & Reference Replacement 2. The viscosities are very comparable with the Acacia Seyal and Acacia Senegal. The dusting powder used was a 50:50 blend of cocoa and flour. Drying times were compared and sealed peanuts were subjected to a sensory test after 2 months of storage in a capped plastic jar.

**TABLE 6:**

| Gum System (40°Brix) | Ave. Viscosity at 25°C, cP | Ave. Viscosity at 60°C, cP |
|---|---|---|
| Gum Arabic, Acacia Seyal | 800 | 225 |
| Gum Arabic, Acacia Senegal | 1040 | 394 |
| Replacement 1 Std. | 799 | 196 |
| Replacement 1 Fast | 914 | 267 |
| Replacement 1 All Natural | 753 | 205 |

Table 7 is directed towards sealing of peanut centers comparing gum arabic and Replacement 1 and Replacement 1 using a batch weight of 1000 g; syrup charge of 10 g; dusting powder-cocoa:flour 50:50 ratio of 10 g. Referring to Table 7, drying times were comparable between gum arabic and Replacement 1 Std. However, Replacement 1 Fast, containing a fast-crystallizing ingredient, shows a significantly faster drying time. Moreover, it protected the peanuts from oxidation and rancidity indicating that it was a better film former.

**TABLE 7:**

| Sealing Syrup | Charge # | Time to Free Flow Minutes | Drying Time to Next Charge Minutes |
|---|---|---|---|
| | | Average of 3 Trials | Average of 3 Trials |
| **40% Gum Arabic Syrup** | | | |
| With Dusting Powder | 1 | 2.50 | 4.33 |
| With Dusting Powder | 2 | 2.11 | 3.19 |
| With Dusting Powder | 3 | 2.39 | 4.67 |
| **Total** | | **7.00** | **12.19** |
| No Dusting Powder | 1 | 2.94 | 4.67 |
| No Dusting Powder | 2 | 2.92 | 4.91 |
| No Dusting Powder | 3 | 3.54 | 6.00 |
| **Total** | | **9.40** | **15.58** |
| **Sensory (after 2 months)** | Rancid smell | | |
| | | | |
| **40% Repl 1 Std Syrup** | | | |
| With Dusting Powder | 1 | 2.29 | 3.69 |
| With Dusting Powder | 2 | 1.97 | 4.94 |
| With Dusting Powder | 3 | 2.24 | 4.22 |
| **Total** | | **6.50** | **12.85** |
| No Dusting Powder | 1 | 3.36 | 5.78 |
| No Dusting Powder | 2 | 3.84 | 5.33 |
| No Dusting Powder | 3 | 3.50 | 5.67 |
| **Total** | | **10.70** | **16.78** |
| **Sensory (after 2 months)** | Slight rancid smell | | |
| **40% Repl 1 FAST Syrup** | | | |
| With Dusting Powder | 1 | 1.53 | 2.83 |
| With Dusting Powder | 2 | 1.50 | 3.06 |
| With Dusting Powder | 3 | 1.70 | 2.78 |
| **Total** | | **4.73** | **8.67** |
| No Dusting Powder | 1 | 1.55 | 3.67 |
| No Dusting Powder | 2 | 1.64 | 4.11 |
| No Dusting Powder | 3 | 2.06 | 4.61 |
| **Total** | | **5.25** | **12.39** |
| **Sensory (after 2 months)** | No rancid smell | | |

**Pastilles Example:** In this example, batches of pastille candies were prepared without gum, with gum arabic (Acacia Seyal), Replacement 1 Std, Replacement 1 Fast and Replacement 1 All Natural using the formulas below. Table 8 describes the formulas for the pastilles.

**TABLE 8:**

| | Control (No Gum) | With Acacia Seyal | With Repl 1 Std. | With Repl 1 Fast | With Repl 1 All Natural |
|---|---|---|---|---|---|
| Sugar | 59.50 | 56.00 | 56.00 | 56.00 | 56.00 |
| Corn Syrup | 25.50 | 24.00 | 24.00 | 24.00 | 24.00 |
| Water | 13.43 | 13.43 | 13.43 | 13.43 | 13.43 |
| Citric Acid | 0.75 | 0.75 | 0.75 | 0.75 | 0.75 |
| Malic Acid | 0.75 | 0.75 | 0.75 | 0.75 | 0.75 |
| Flavor | 0.07 | 0.07 | 0.07 | 0.07 | 0.07 |
| Gum System | 0.00 | 5.00 | 5.00 | 5.00 | 5.00 |

In this example, water and corn syrup were first added to the cooking pan. Sugar and gum system were dry- blended and added to the batch while mixing. Then the mixture was heated to about 148 to 150°C, heat was turned off, and acids and flavor were added. While still liquid and hot, the candy syrup was poured into molds. When candies crystallized, they were placed in the humidity chamber overnight at about 12% relative humidity (RH), packed and refrigerated to prevent sweating. Hardness of the candies was measured using TA XT Texture Analyzer.

Replacement 1 Std., Replacement 1 Fast and Replacement 1 All Natural increased the hardness of the candy by the same magnitude or even slightly higher than the gum arabic (Acacia Seyal) over that of the control with no gums added. In terms of color, gum arabic seemed to brown more than the Replacements. Table 9 includes hardness values of the pastille cubes made with gum arabic and the Replacements compared to the control candy with sugar only; average of 2 trials and 6 to 20 replications per trial.

The hardness of the candies in terms of grams force needed to crack were input into the table. Referring to Table 9, the control candy with sugar alone and no gums has the lowest hardness value. Candies made with Replacement 1 Std. and Replacement 1 Fast showed comparable or slightly higher hardness values compared to gum arabic.

**TABLE 9:**

| Pastille Samples | Force 1 | Distance 1 |
|---|---|---|
| | g | mm |
| Control [no gum, sugar only] | | |
| Average | 11396 ± 1552 | 2.778 |
| With Gum Arabic | | |
| Average | 12436 ± 2098 | 2.372 |
| With Replacement 1 Std | | |
| Average | 12886 ± 2173 | 2.468 |
| With Replacement 1 Fast | | |
| Average | 13336 ± 2469 | 2.084 |
| With Replacement 1 All Natural | | |
| Average | 12904 ± 2353 | 2.669 |

Although the disclosure has been described and illustrated with a certain degree of particularity, it is understood that the disclosure has been made only by way of example, and that numerous changes in the conditions and order of steps can be resorted to by those skilled in the art.

## Claims

1. Use of a gum arabic replacement, partial replacement or extending composition in sugar and sugar free panning, confections and food adhesion applications, wherein the gum arabic replacement, partial replacement or extending composition is prepared by a method comprising the steps of:
a. blending a strong (key) film former selected from the group consisting of cellulose gum, methyl cellulose, hydroxypropyl methylcellulose (HPMC), propylene glycol alginate, carrageenan, pectin, alginate, gellan, agar, konjac, xanthan, fenugreek, tara, and combinations thereof between 0.1% and 10% of a total composition with,
b. a low viscosity cracking agent selected from the group consisting of maltodextrin, monosaccharides, disaccharides, oligosaccharides, larch gum, polydextrose, and combinations thereof;
c. a tackiness modifier selected from the group consisting of starch, guar gum, locust bean gum and combinations thereof;
d. a fast-crystallizing agent selected from the group consisting of inulin, erythritol, low DE maltodextrin, lactose, and combinations thereof;
wherein the composition has a viscosity at concentration of 35°- 40° Brix being 10 cP to 2000 cP at 25°C and a film cast at 5 mils to 10 mils cracks into flakes on drying that lies between that of materials that can generate continuous films and materials that crystallize and do not form films at all.

## Patentansprüche

1. Verwendung eines Gummi-arabicum-Ersatzes, -Teilersatzes oder einer - Streckzusammensetzung bei Anwendungen bei Zucker- und zuckerfreiem Überzug, Konfekten und Haftvermögen von Nahrungsmitteln, wobei der Gummi-arabicum-Ersatz, -Teilersatz oder die -Streckzusammensetzung durch ein Verfahren hergestellt wird, das die folgenden Schritte aufweist:
a. Vermischen eines starken (Schlüssel-)Filmbildners, der aus der Gruppe ausgewählt wird, die besteht aus Cellulosegummi, Methylcellulose, Hydroxypropylmethylcellulose (HPMC), Propylenglycolalginat, Carrageenan, Pectin, Alginat, Gellan, Agar, Konjac, Xanthan, Bockshornklee, Tara, und Kombinationen davon zwischen 0,1% und 10% einer Gesamtzusammensetzung mit
b. einem niederviskosen Rissbildungsmittel, das aus der Gruppe ausgewählt, die besteht aus Maltodextrin, Monosacchariden, Disacchariden, Oligosacchariden, Lärchengummi, Polydextrose und Kombinationen davon;
c. einem Klebkraft-Modifikator, der aus der Gruppe ausgewählt wird, die besteht aus Stärke, Guargummi, Johannisbrotgummi und Kombinationen davon;
d. einem schnell kristallisierenden Mittel, das aus der Gruppe ausgewählt wird, die besteht aus Inulin, Erythritol, Niedrig-DE-Maltodextrin, Lactose und Kombinationen davon;
wobei die Zusammensetzung eine Viskosität bei einer Konzentration von 35°- 40° Brix hat, die 10 cP bis 2000 cP bei 25°C beträgt, und ein bei 5 mil bis 10 mil gegossener Film sich beim Trocknen in Flocken spaltet, das zwischen dem von Materialien, die kontinuierliche Filme erzeugen können, und Materialien liegt, die kristallisieren und keine Filme bilden.

## Revendications

1. Utilisation d'une composition de remplacement, remplacement partiel ou extension de gomme arabique dans des applications de dragées, confiserie et adhésion alimentaire avec et sans sucre, dans laquelle la composition de remplacement, remplacement partiel ou extension de gomme arabique est préparée par un procédé comprenant les étapes de :
a. mélange d'un agent filmogène fort (principal) choisi dans le groupe constitué de gomme de cellulose, méthylcellulose, hydroxypropylméthylcellulose (HPMC), alginate de propylène glycol, carraghénine, pectine, alginate, gellane, gélose, konjac, xanthane, fenugrec, tara, et des combinaisons de ceux-ci entre 0,1 % et 10 % d'une composition totale avec,
b. un agent de craquelure à viscosité faible choisi dans le groupe constitué de maltodextrine, monosaccharides, disaccharides, oligosaccharides, gomme de mélèze, polydextrose, et des combinaisons de ceux-ci ;
c. un modificateur d'adhésivité choisi dans le groupe constitué de l'amidon, la gomme de guar, la gomme de carouble et des combinaisons de ceux-ci ;
d. un agent à cristallisation rapide choisi dans le groupe constitué de l'inuline, l'érythritol, la maltodextrine à DE faible, le lactose, et des combinaisons de ceux-ci ;
dans laquelle la composition a une viscosité à une concentration de 35° à 40° Brix étant de 10 cP à 2000 cP à 25 °C et un film coulé à 5 mil à 10 mil se craquelle en lamelles lors du séchage, qui est située entre celle de matériaux qui peuvent générer des films continus et des matériaux qui cristallisent et ne forment aucun film.
